(19) Europäisches Patentamt
European Patent Office
Office européen des brevets

(11) Veröffentlichungsnummer: **0 163 606**
**B1**

# (12) EUROPÄISCHE PATENTSCHRIFT

(45) Veröffentlichungstag der Patentschrift: **06.09.89**

(51) Int. Cl.⁴: **C 07 D 249/08**, C 07 D 233/56, A 01 N 43/653, A 01 N 43/50

(21) Anmeldenummer: **85810231.2**

(22) Anmeldetag: **15.05.85**

(54) **Mikrobizide 1-Fluor-1-azolyl-2,2-diarylethanderivate.**

(30) Priorität: **23.05.84 CH 2534/84**

(43) Veröffentlichungstag der Anmeldung: **04.12.85 Patentblatt 85/49**

(45) Bekanntmachung des Hinweises auf die Patenterteilung: **06.09.89 Patentblatt 89/36**

(84) Benannte Vertragsstaaten: **AT BE CH DE FR GB IT LI NL**

(56) Entgegenhaltungen:
**EP-A- 0 004 315**
**EP-A- 0 008 057**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Frick, Willy, Dr., Alter Kirchweg 15, CH-4148 Pfeffingen (CH)**
Erfinder: **Meyer, Alfred, Dr., St. Galler-Ring 220, CH-4054 Basel (CH)**
Erfinder: **Nyfeler, Robert, Dr., Bärenfelserstrasse 8, CH-4057 Basel (CH)**

**EP 0 163 606 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue 1-Halo-1-azolyl-2,2-diaryl-ethan-derivate der nachstehenden Formel I deren Säureadditionssalze und Metallkomplexe. Die Erfindung betrifft ferner die Herstellung dieser Substanzen sowie mikrobizide Mittel, die als Wirkstoff mindestens eine dieser Verbindungen enthalten. Die Erfindung betrifft auch die Verwendung der Wirkstoffe zur Bekämpfung schädlicher Mikroorganismen, insbesondere Pilze.

Die erfindungsgemässen Verbindungen haben die Formel I

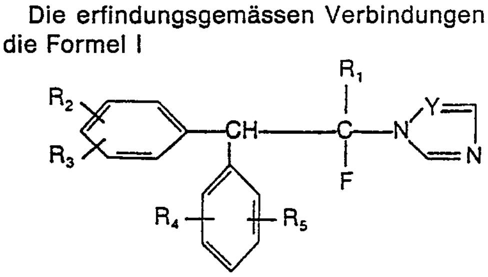

(I)

worin

Y für –CH= oder –N= steht; $R_1$ Wasserstoff oder $C_1$–$C_3$-Alkyl bedeutet; und $R_2$, $R_3$, $R_4$ und $R_5$ unabhängig voneinander für Wasserstoff, Halogen, Methyl, Methoxy, $CF_3$, $OCF_3$, $OCHF_2$, Cyano und/oder unsubstituiertes oder durch Fluor, Chlor und/oder Brom substituiertes Phenoxy stehen; und schließen die Säureadditionssalze und Metallkomplexe mit ein.

Halogen ist Fluor, Chlor, Brom und Jod. $C_1$–$C_3$-Alkyl bedeutet Methyl, Ethyl, Propyl und Isopropyl.

Beispiele salzbildender Säuren sind anorganische Säuren; Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, Phosphorige Säure, Salpetersäure und organische Säuren wie Essigsäure, Trifluoressigsäure, Trichloressigsäure, Propionsäure, Weinsäure, Glykolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure. Diese Säuren werden nach an sich bekannten Methoden an die freien Verbindungen der Formel I addiert.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, von Mangan, Eisen, Kobalt, Nickel, Zirkonium, Kupfer, Zink.

Die Verbindungen der Formel I sind bei Raumtemperatur stabil. Sie lassen sich auf dem Agrarsektor oder verwandten Gebieten präventiv und kurativ zur Bekämpfung von pflanzenschädigenden Mikroorganismen einsetzen, dabei sind die Triazolylmethylderivate im Umfang der Formel I bevorzugt. Die erfindungsgemässen Wirkstoffe der Formel I zeichnen sich in weiten Anwendungskonzentrationen durch eine sehr gute fungizide Wirkung und problemlose Anwendung aus.

Aufgrund ihrer ausgeprägten mikrobiziden Wirkung sind folgende Substanzgruppen bevorzugt:

a) Verbindungen der Formel I, worin Y für –N= steht; $R_1$ Wasserstoff oder $C_1$–$C_3$-Alkyl bedeutet; $R_2$ für Wasserstoff, 2–F, 2–Cl, 2–$CH_3$, 2–$C_2H_5$ oder 2–$CF_3$ steht; $R_3$ für Wasserstoff, 4–Cl, 4–$OCHF_2$, 4-Phenoxy oder 4-(4'-Halophenoxy) steht; $R_4$ Wasserstoff, 2–F, 2–Cl, 2–$CH_3$, 2–$C_2H_5$ oder 2–$CF_3$ bedeutet; und $R_5$ für Wasserstoff, 4–Cl, 4–F, 4–$OCHF_2$, 4-Phenoxy oder 4-(4'-Halophenoxy) steht.

b) Verbindungen der Formel I, worin Y für –N= steht; $R_1$ Wasserstoff bedeutet, $R_2$ für Wasserstoff, 2–Cl oder 2–$CH_3$ steht; $R_3$ p-Halophenoxy bedeutet, $R_4$ Wasserstoff ist und $R_5$ für p-Halogen steht.

Als Einzelsubstanzen sind z. B. folgende Verbindungen der Formel I besonders bevorzugt:

1-Fluor-1-(1H-1,2,4-triazol-1-yl)-2-(2,4-dichlorphenyl)-2-(4-fluorphenyl)ethan;

1-Fluor-1-(1H-1,2,4-triazol-1-yl)-1-methyl-2-(2,4-dichlorphenyl)-2-(4-fluorphenyl)ethan;

1-Fluor-1-(1H-1,2,4-triazol-1-yl)-1-ethyl-2-(2,4-dichlorphenyl)-2-(4-fluorphenyl)ethan;

1-Fluor-1-(1H-imidazol-1-yl)-2-(2,4-dichlorphenyl)-2-(4-fluorphenyl)ethan;

1-Fluor-1-(1H-1,2,4-triazol-1-yl)-2-bis(4-chlorphenyl)ethan;

1-Fluor-1-(1H-1,2,4-triazol-1-yl)-2-(4-chlorphenyl)-2-(4-fluorphenyl)ethan;

1-Fluor-1-(1H-1,2,4-triazol-1-yl)-2-(4-chlorphenyl)-2-(2-fluorphenyl)ethan;

1-Fluor-1-(1H-1,2,4-triazol-1-yl)-2-phenyl-2-(2,4-dichlorphenyl)ethan.

Die Verbindungen der Formel I lassen sich dadurch erhalten, daß man einen Alkohol der Formel II

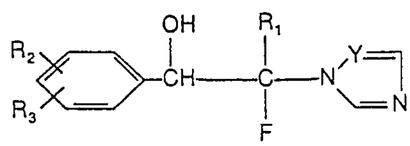

(II)

in Gegenwart einer Säure oder Lewis-Säure mit einem Benzolderivat der Formel III

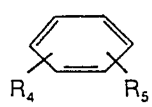

(III)

umsetzt, wobei $R_1$, $R_2$, $R_3$, $R_4$, $R_5$ und Y die unter Formel I angegebenen Bedeutungen haben. Die Reaktion wird vorteilhaft im Temperaturbereich von −20 °C bis +80 °C, bevorzugt −10 °C bis +30 °C, besonders bevorzugt −5 °C bis +20 °C durchgeführt.

Als Lösungsmittel dient vorzugsweise ein Überschuß des Reaktanden der Formel III. Als reaktionsinerte Verdünnungsmittel können z. B. Nitrobenzol oder aliphatische Kohlenwasserstoffe wie Cyclohexan, Hexan usw. anwesend sein. Geeignete Säuren sind z. B. konzentrierte Mineralsäuren, wie Schwefelsäure, Phosphorsäure oder Halogenwasserstoffsäuren (HCl, HF, HBr) und Sulfosäuren wie Benzolsulfonsäure oder p-Toluolsulfonsäure. Als Lewis-Säuren eignen sich z. B. die Halogenide des Bors, Aluminiums, Zinks, Antimons, Quecksilbers, Kupfers, Silbers oder Titans ($BF_3$, $ZnCl_2$, $AlCl_3$, $FeCl_3$, $SbCl_5$, $SnCl_4$, $TiCl_4$, $ZnCl_2$ usw.). Besonders bevorzugt sind konzentrierte $H_2SO_4$ und $AlCl_3$.

Die Ausgangsverbindungen der Formel II sind aus den zugrundeliegenden α,α-Dihalogenketonen der Formel IV

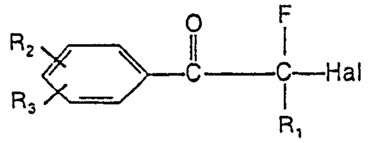 (IV)

durch Reaktion mit einem Azol der Formel V

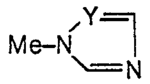 (V)

zugänglich, wobei $R_1$, $R_2$, $R_3$ und Y die unter Formel I angegebenen Bedeutungen haben, Hal für Halogen, vorzugsweise Chlor oder Brom steht und Me Wasserstoff oder vorzugsweise ein Alkali- oder Erdalkalimetallkation, insbesondere $Na^+$ oder $K^+$, darstellt. Derartige Reaktionen – Ersatz eines Halogenatoms durch einen Azolring – sind aus der Literatur hinlänglich bekannt.

Die Verbindungen der Formel IV sind durch Halogenierung der zugrundeliegenden, bekannten α-Halogenketone der Formel VI;

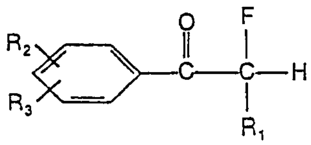 (VI)

worin die Substituenten wie unter Formel I definiert sind, herstellbar [F. Bergmann et al., J. Am. Chem. Soc. 79, 4178 (1957)].

Das beschriebene Herstellungsverfahren ist ein Bestandteil dieser Erfindung.

Die Verbindungen der Formel I besitzen in Nachbarstellung zur Azolgruppe stets ein asymmetrisches *C-Atom und können daher in zwei enantiomeren Formen vorliegen. Im allgemeinen entsteht bei der Herstellung dieser Substanzen ein Gemisch beider Enantiomeren. Dieses läßt sich auf übliche Weise, z. B. durch fraktionierte Kristallisation von Salzen mit optisch aktiven, starken Säuren, in die reinen optischen Antipoden aufspalten. Beide Enantiomeren können unterschiedlich biologische Aktivitäten aufweisen. In den Fällen in denen $R_2$ und $R_3$ andere Bedeutungen haben als $R_4$ und $R_5$, tritt in Nachbarschaft zu den beiden Phenylringen ein weiteres Asymmetriezentrum auf, das zur Existenz von diastereomeren Gemischen (threo- und erythro-Form) führt. Die Diastereomeren können mit Hilfe üblicher physikalischer Methoden getrennt werden.

Die vorliegende Erfindung betrifft alle reinen Enantiomeren bzw. Diastereomeren und deren Gemische untereinander.

In der veröffentlichten euopäischen Anmeldung 004 315 werden 1,1-Diphenylätherderivate als Mikrobizide, z. B. auch zur Bekämpfung phytopathogener Fungi, beschrieben.

Es wurde überraschend festgestellt, daß Verbindungen der Formel I ein für praktische Bedürfnisse sehr günstiges Mikrobizid-Spektrum gegen Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z. B. Venturia, Podosphaera, Erysiphe, Monilinia, Uncinula); Basidiomyceten (z. B. die Gattungen Hemileia, Rhizoctonia, Puccinia); Fungi imperfecti (z. B. Botrytis, Helmintosporium, Fusarium, Septoria, Cercospora und Alternaria). Überdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemäßen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung pathogener Mikroorganismen, insbesondere pflanzenschädigender Pilze bzw. die präventive Verhütung eines Befalls an Pflanzen.

Als Zielkulturen für die hierin offenbarten Indikationsgebiete gelten im Rahmen dieser Erfindung z. B. folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat; Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Die Verbindungen der Formel I werden dabei in unveränderter Form oder vorzugsweise zusammen mit den in der Formulierungstechnik üblichen Hilfsmittel eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren werden gleich wie die Art der Mittel den angestrebten Zielen und den gegebenen Verhältnissen entsprechend gewählt. Im Agrarsektor liegen günstige Aufwandmengen im allgemeinen bei 50 g bis 5 kg Aktivsubstanz (AS) je ha; bevorzugt 100 g bis 2 kg AS/ha, insbesondere bei 100 g bis 600 g AS/ha.

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnußöl oder Sojaöl; oder Wasser.

Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z. B. Phosphatidylethanolamin, Phosphatidylserin, Phosphatidylglycerin oder Lysolecithin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u. a. in folgenden Publikationen beschrieben:

«Mc Cutcheon's Detergents and Emulsifiers Annual»
BC Publishing Corp., Ringwood New Jersey, 1981;

Helmut Stache «Tensid-Taschenbuch» Carl Hanser-Verlag
München/Wien 1981.

M. and J. Ash. «Encyclopedia of Surfactants», Vol. I–III, Chemical Publishing Co., New York, 1980–1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht.

Herstellungsbeispiele
Beispiel H1:
Herstellung von

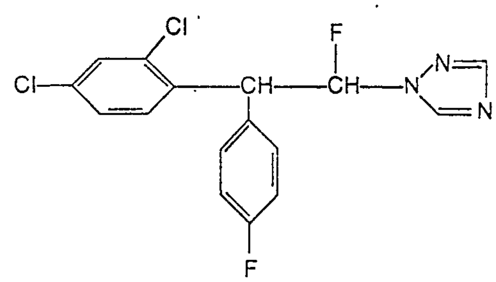

(1.1)

1-Fluor-1-(1H-1,2,4-triazol-1-yl)-2-(2,4-dichlorphenyl)-2-(4-fluorphenyl)ethan

a) Herstellung des Ausgangsproduktes

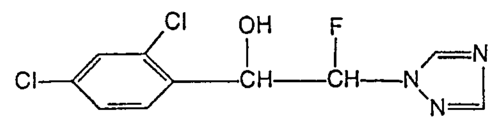

1-(2,4-Dichlorphenyl)-2-fluor-2-(1H-1,2,4-triazol-1-yl)ethanol

Zu einer Suspension von 2,4 g 1-(2,4-Dichlorphenyl)-2-fluor-2-(1H-1,2,4-triazol-1-yl)ethanon in 9,5 ml Methanol und 0,5 ml Wasser werden unter Rühren bei 5–10 °C 0,12 g Natriumborhydrid eingetragen und die resultierende Mischung zuerst 1 Stunde bei 0–5 °C und dann 2 Stunden bei Raumtemperatur gerührt, wobei allmählich eine klare Lösung entsteht. Diese Lösung wird dann auf Eiswasser gegossen und das Gemisch zweimal mit Dichlormethan extrahiert. Die vereinigten Extrakte werden über Natriumsulfat getrocknet, filtriert und eingeengt. Nach Hochvakuumdestillation des Rückstands erhält man den obigen Alkohol als Diastereomerengemisch. Sdp. des Rohproduktes 150–160 °C/0,07 mbar. Nach Kristallisation aus Diethylether/Diisopropylether erhält man das gereinigte Diastereomerengemisch in Form beiger Kristalle. Schmelzintervall 103–112 °C.

b) Herstellung des Endproduktes

In ein auf 0 °C gekühltes Gemisch von 1 Liter 98%iger $H_2SO_4$ und 300 ml Fluorbenzol werden unter kräftigem Rühren 276 g 1-(2,4-Dichlorphenyl)-2-fluor-2-(1H-1,2,4-triazol-1-yl)ethanol derart eingetragen, daß die Innentemperatur +10 °C nicht übersteigt. Die Reaktionsmischung wird anschließend noch 6 Stunden bei Raumtemperatur gerührt, sodann auf ca. 1,5 kg Eis gegossen und dreimal mit je 300 ml Methylenchlorid extrahiert. Die vereinigten Extrakte werden mit Wasser neutral gewaschen, über Natriumsulfat getrocknet, filtriert und überschüssiges Lösungsmittel und Fluorbenzol abdestilliert. Das resultierende hellbraune Harz wird bei 0,05 mbar und 160 °C (Badtemperatur) einer Kurzwegdestillation unterworfen. Man erhält 300 g (Ausbeute ca. 85%) einer hellen glasartigen Masse. Mittels Cyclohexan/Diethylether kann dieses Glas zur Kristallisation gebracht werden. Schmelzintervall 96–104 °C. Die analytischen Daten zeigen, daß es sich um ein Diastereomerengemisch der Verbindung Nr. 1.1 handelt.

Analog zu den beschriebenen Arbeitsweisen lassen sich auch die nachfolgend aufgeführten Verbindungen der Formel I herstellen, die, sofern nicht speziell erwähnt, als Diastereomerengemische anfallen:

Tabelle 1: Verbindungen der Formel I

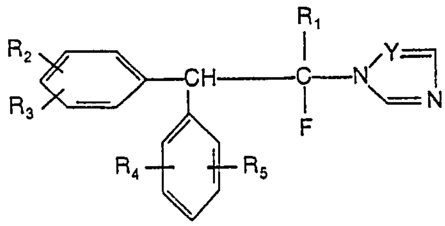

(I)

| Verb. Nr. | $R_1$ | $R_2$ | $R_3$ | $R_4$ | $R_5$ | Y | physik. Konst. [°C] |
|---|---|---|---|---|---|---|---|
| 1.1 | H | 2-Cl | 4-Cl | H | 4-F | N | Smp. 96–104° |
| 1.2 | H | 2-Cl | 4-Cl | H | 4-F | CH | Öl |
| 1.3 | $CH_3$ | 2-Cl | 4-Cl | H | 4-F | N | Smp. 101–103° |
| 1.4 | $C_2H_5$ | 2-Cl | 4-Cl | H | 4-F | N | Smp. 99–101° |
| 1.5 | H | 2-Cl | 4-Cl | H | H | N | $n_D^{50}$ 1.5764 |
| 1.6 | $CH_3$ | 2-Cl | 4-Cl | H | H | N | Smp. 84–86° |
| 1.7 | H | 2-Cl | 4-Cl | 2-Cl | H | N | |
| 1.8 | $CH_3$ | 2-Cl | 4-Cl | 2-Cl | H | N | |
| 1.9 | H | 2-Cl | 4-Cl | H | 4-Cl | N | $n_D^{50}$ 1.5910 |
| 1.10 | $CH_3$ | 2-Cl | 4-Cl | H | 4-Cl | N | $n_D^{50}$ 1.5795 |
| 1.11 | $C_2H_5$ | 2-Cl | 4-Cl | H | 4-Cl | N | |
| 1.12 | $CH_3$ | 2-Cl | 4-Cl | H | 4-Br | N | Smp. 107–108° |
| 1.13 | H | 2-Cl | 4-Cl | 2-F | H | N | |
| 1.14 | H | H | 4-Cl | 2-F | H | N | $n_D^{50}$ 1.5556 |
| 1.15 | $C_3H_7$-n | 2-Cl | 4-Cl | 2-F | H | N | |
| 1.16 | H | 2-Cl | 4-Cl | 2-$OCHF_2$ | H | N | |
| 1.17 | H | 2-Cl | 4-Cl | H | 4-$OCF_3$ | N | |
| 1.18 | H | 2-Cl | H | H | 4-Cl | N | |
| 1.19 | $CH_3$ | 2-Cl | H | H | 4-Cl | N | |
| 1.20 | H | 2-Cl | H | H | 4-F | N | |
| 1.21 | H | 2-Cl | H | H | 4-F | CH | |
| 1.22 | $CH_3$ | 2-Cl | H | H | 4-F | N | |
| 1.23 | H | 2-F | H | H | 4-F | N | $n_D^{50}$ 1.5395 |
| 1.24 | $CH_3$ | 2-F | H | H | 4-F | N | |
| 1.25 | $C_2H_5$ | 2-F | H | H | 4-F | N | |
| 1.26 | H | H | 4-Cl | H | 4-Cl | N | Smp. 74–75° |
| 1.27 | $CH_3$ | H | 4-Cl | H | 4-Cl | N | |
| 1.28 | H | H | 4-Cl | H | 4-F | N | $n_D^{50}$ 1.5630 |
| 1.29 | H | 2-$CH_3$ | 4-Cl | H | 4-Cl | N | |
| 1.30 | $CH_3$ | 2-$CH_3$ | 4-Cl | H | 4-Cl | N | |
| 1.31 | H | 2-Cl | 4-$OCHF_2$ | H | 4-Cl | N | |
| 1.32 | H | H | 4-Cl | H | 4-Br | N | Smp. 79–80° |
| 1.33 | H | 2-$CF_3$ | H | H | 4-Cl | N | |
| 1.34 | H | 2-Cl | 4-Cl | 2-$CF_3$ | H | N | |
| 1.35 | $CH_3$ | 2-Cl | 4-Cl | H | 4-CN | N | |

Tabelle 2: Verbindungen der Formel

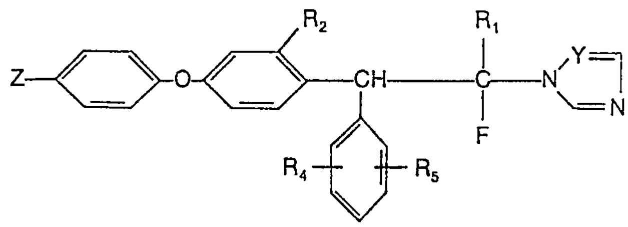

| Verb. Nr. | $R_1$ | $R_2$ | Z | $R_4$ | $R_5$ | Y | physik. Konst. [°C] |
|---|---|---|---|---|---|---|---|
| 2.1 | H | H | Cl | H | H | N | $n_D^{50}$ 1.5758 |
| 2.2 | $CH_3$ | H | Cl | H | H | N | |
| 2.3 | $CH_3$ | H | Cl | H | 4-F | N | |
| 2.4 | $CH_3$ | H | Cl | H | 4-F | CH | |
| 2.5 | H | H | Br | H | 4-Cl | N | |
| 2.6 | H | H | F | 2-Cl | 4-Cl | N | |
| 2.7 | H | $CH_3$ | Cl | H | H | N | |
| 2.8 | $CH_3$ | $CH_3$ | Cl | H | H | N | Smp. 125–127° |
| 2.9 | H | $CH_3$ | Cl | H | 4-F | N | Smp. 82–84° |
| 2.10 | $CH_3$ | $CH_3$ | Cl | H | 4-F | N | Smp. 95–96° |
| 2.11 | $C_2H_5$ | $CH_3$ | Cl | H | 4-F | N | |
| 2.12 | H | $CH_3$ | Cl | H | 4-Cl | N | |
| 2.13 | $CH_3$ | $CH_3$ | Cl | H | 4-Cl | N | |
| 2.14 | H | $C_2H_5$ | H | H | 4-F | N | |
| 2.15 | $CH_3$ | $C_2H_5$ | H | H | 4-F | N | |
| 2.16 | H | H | H | H | H | N | |
| 2.17 | $CH_3$ | H | H | H | H | N | |
| 2.18 | H | Cl | H | H | 4-F | N | |
| 2.19 | $CH_3$ | Cl | H | H | 4-F | N | |
| 2.20 | H | Cl | Cl | H | H | N | |
| 2.21 | H | Cl | Cl | H | 4-F | N | $n_D^{50}$ 1.5761 |
| 2.22 | $CH_3$ | Cl | Cl | H | 4-F | N | |
| 2.23 | $C_2H_5$ | Cl | Cl | H | 4-F | N | |
| 2.24 | H | H | Cl | H | 4-Cl | N | Smp. 95–97° |
| 2.25 | $CH_3$ | Cl | Cl | H | 4-Cl | N | |
| 2.26 | H | Cl | F | H | 4-Cl | N | |

Formulierungsbeispiele für Wirkstoffe der Formel I (% = Gewichtsprozent)

| F1. Lösungen | a) | b) | c) | d) |
|---|---|---|---|---|
| Wirkstoff aus den Tabellen | 80% | 10% | 5% | 95% |
| Ethylenglykol-mono-methyl-ether | 20% | – | – | – |
| Polyethylenglykol MG 400 | – | 70% | – | – |
| N-Methyl-2-pyrrolidon | – | 20% | – | – |
| Epoxydiertes Kokosnußöl | – | – | 1% | 5% |
| Benzin (Siedegrenzen 160–190 °C) | – | – | 94% | – |

(MG = Molekulargewicht)

Die Lösungen sind zur Anwendung in Form kleinster Tropfen geeignet.

| F2. Granulate | a) | b) |
|---|---|---|
| Wirkstoff aus den Tabellen | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschließend im Vakuum abgedampft.

| F3. Stäubemittel | a) | b) |
|---|---|---|
| Wirkstoff aus den Tabellen | 2% | 5% |
| Hochdisperse Kieselsäure | 1% | 5% |
| Talkum | 97% | – |
| Kaolin | – | 90% |

Durch inniges Vermischen auf Trägerstoffe mit dem Wirkstoff erhält man gebrauchsfertige Stäubemittel.

| F4. Spritzpulver | a) | b) | c) |
|---|---|---|---|
| Wirkstoff aus den Tabellen | 25% | 50% | 75% |
| Na-Ligninsulfoant | 5% | 5% | – |
| Na-Laurylsulfat | 3% | – | 5% |
| Na-Diisobutylnaphthalin-sulfonat | – | 6% | 10% |
| Octylphenolpolyethylenglykol-ether (7–8 Mol Ethylenoxid) | – | 2% | – |
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F5. Emulsions-Konzentrat | |
|---|---|
| Wirkstoff aus den Tabellen | 10% |
| Octylphenolpolyethylenglykolether (4–5 Mol Ethylenoxid) | 5% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| F6. Umhüllungs-Granulat | |
|---|---|
| Wirkstoff aus den Tabellen | 3% |
| Polyethylenglykol (MG 200) | 3% |
| Kaolin | 94% |

(MG = Molekulargewicht

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

Biologische Beispiele:
Beispiel B1:
Wirkung gegen Puccinia graminis auf Weizen
Residual-protektive Wirkung

Weizenpflanzen wurden 6 Tage nach der Aussaat mit einer aus Spritzpulver des Wirkstoffes hergestellten Spritzbrühe (0,02% Aktivsubstanz) besprüht. Nach 24 Stunden wurden die behandelten Pflanzen mit einer Uredosporensuspensionen des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Unbehandelte aber infizierte Kontrollpflanzen zeigten einen Puccinia-Befall von 100%. Verbindungen aus den Tabellen 1 und 2 zeigten gegen Puccinia-Pilze eine gute Wirkung. Unter anderen hemmten die Verbindungen Nr. 1.1, 1.3, 1.4, 1.5, 1.10, 1.28 den Puccinia-Befall auf 0 bis 5%.

Beispiel B2:
Wirkung gegen Cercospora arachidicola auf Erdnußpflanzen
a) Residual-protektive Wirkung

10–15 cm hohe Erdnußpflanzen wurden mit einer aus Spritzpulver der Wirksubstanz hergestellten Spritzbrühe (0,02% Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschließend bis zum Auftreten der typischen Blattflecken in einem Gewächshaus aufgestellt. Die Beurteilung der fungiziden Wirkung erfolgte 12 Tage nach der Infektion basierend auf Anzahl und Größe der auftretenden Flecken.

b) Systemische Wirkung

Zu 10–15 cm hohen Erdnußpflanzen wurde eine aus Spritzpulver des Wirkstoffes hergestellte Spritzbrühe gegossen (0,06% Aktivsubstanz bezogen auf das Erdvolumen). Nach 48 Stunden wurden die behandelten Pflanzen mit einer Konidiensuspension des Pilzes infiziert und 72 Stunden bei ca. 21 °C und hoher Luftfeuchtigkeit inkubiert. Anschließend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Größe der Flecken = 100%), zeigten Erdnußpflanzen, die mit Wirkstoffen aus den Tabellen 1 und 2 behandelt wurden, einen stark reduzierten Cercospora-Befall. So verhinderten die Verbindungen Nr. 1.1, 1.3, 1.4, 1.5, 1.14, 1.26, 1.28, 2.1, 2.8, 2.9 und 2.24 in obigen Versuchen das Auftreten von Flecken fast vollständig (0–10%).

Beispiel B3:
Wirkung gegen Erysiphae graminis auf Gerste
a) Residual-protektive Wirkung

Ca. 8 cm hohe Gerstenpflanzen wurden mit einer aus Spritzpulver des Wirkstoffes hergestellten Spritzbrühe (0,02% Aktivsubstanz) besprüht. Nach 3–4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, daß die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100%. Unter anderen Verbindungen aus den Tabellen 1 und 2 hemmten die Verbindungen Nr. 1.1, 1.3, 1.4, 1.5, 1.6, 1.9, 1.10, 1.12, 1.14, 1.23, 1.26, 1.28, 1.32; 2.1, 2.8, 2.9, 2.10, 2.21 und 2.24 den Pilzbefall bei Gerste auf 0 bis 5%.

**Beispiel B4:**
**Residual-protektive Wirkung gegen Venturia inaequalis auf Apfeltrieben**

Apfelstecklinge mit 10–20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90–100% relativer Luftfeuchtigkeit inkubiert und während 10 weiteren Tagen in einem Gewächshaus bei 20–24 °C aufgestellt. Der Schorfbefall wurde 15 Tage nach der Infektion beurteilt. Verbindungen aus den Tabellen 1 und 2 bewirkten eine deutliche Hemmung des Krankheitsbefalls. So hemmten die Verbindungen 1.1, 1.3, 1.4, 1.5, 1.6, 1.9, 1.10, 1.12, 1.14, 1.23, 1.26, 1.28, 1.32; 2.1, 2.8, 2.9, 2.10, 2.21 und 2.24 den Pilzbefall fast vollständig (0–5%). Unbehandelte aber infizierte Triebe zeigten einen 100%igen Venturia-Befall.

Beispiel B5:
Wirkung gegen Botrytis cinerea auf Bohnen
Residual protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95–100% relativer Luftfeuchtigkeit und 21 °C erfolgte die Beurteilung des Pilzbefalls. Der Botrytis-Befall unbehandelter aber infizierter Bohnenpflanzen betrug 100%. Der Befall nach Behandlung mit einer der Verbindungen der Formel I betrug <20%; bei Behandlung mit den Verbindungen Nr. 1.1, 1.5, 1.28 und anderen trat kein Befall auf (0–5%).

Hervorzuheben ist die ausgezeichnete Wirkung der Verbindungen der Formel I und ihrer Salze gegen Candidiasis und andere Infektionen durch Pilze, insbesondere Dermatophyten wie Trichophyton, Blastomyces, Torulopsis, Microsporum, Sporotrichum, Aspergillus, Staphylococcus und andere. In geeigneten Formulierungen kann die Zubereitung fest, halbfest oder flüssig sein und in Form von Tabletten, Kapseln, Pulvern, Suppositorien, fließenden Lösungen, Suspensionen, Cremen, Lotionen, Gelen, Salben und dergleichen vorliegen. Nicht toxische Träger, die normalerweise für feste Formulierungen verwendet werden, sind beispielsweise Tricalciumphosphat, Calciumcarbonat, Kaolin, Bentonit, Talc, Gelatine, Lactose, Stärke und dergleichen; für halbfeste Formulierungen, beispielsweise Polyalkylenglykole, Vaseline, Petrolatum und andere Cremebasen; für flüssige Zubereitungen, beispielsweise Wasser, Öle vegetabilen Ursprungs und niedrig siedende Lösungsmittel wie Isopropanol.

Für die vorgenannten Anwendungszwecke können die beschriebenen Verbindungen der Formel I und deren antimikrobielle Säureadditionssalze bzw. solche Wirksubstanz enthaltende Zubereitungen Menschen und Tieren nach konventionellen Methoden verabreicht werden, beispielsweise örtlich, oral, parenteral, und dergleichen. Örtliche Verabreichung umfaßt auch intravaginale Anwendung.

Für derartige Behandlungen kann ein Flächenbereich, der von Pilz- oder Bakterienwachstum befallen ist oder gegen derartigen Befall geschützt werden soll, mit Verbindungen der Formel I oder deren antimikrobiellen Säureadditionssalzen oder solche Wirksubstanzen enthaltenden Zubereitungen behandelt werden, beispielsweise durch Bestäuben, Beträufeln, Besprühen, Spülen, Beschichten.

Für systemische, wie orale oder parenterale, Verabreichung zur Erzielung wirksamer Resultate ist es zweckmäßig, die Wirksubstanz in einer Tagesdosierung von 1–100 mg/kg Körpergewicht, vorzugsweise 5–50 mg/kg Körpergewicht, vorzugsweise in mehrere Gaben zu verabreichen. Für lokale Verabreichung, wird jedoch proportional weniger Aktivsubstanz benötigt.

Beispiel B-6:
Bestimmung von Pilz-Hemmkonzentrationen (MIC)

36 g Mycophil-Agar (bestehend aus 10 g Pepton, 10 g Dextrose und 16 g Agar) werden in 1000 ml dest. Wasser suspendiert. Je 20 ml der verdünnten Suspension werden in Petrischalen (∅ 85 cm) gegeben und bei 35 °C trocknen gelassen. Danach wird jede Agarplatte in der Schale mit einer Wirkstofflösung (Lösungsmittel Dimethylsulfoxid) mit Hilfe eines Stifts eines Steer-Applikators versetzt, so daß Wirkstoffkonzentrationen von 128, 64, 32, 16, 8, 4, 2 bzw. 1 mcg/ml entstehen. Jede dieser Platten wird nun an verschiedenen Stellen mit den Mikroorganismen beimpft (Tropfengröße 10 µl enthaltend $10^3$ Keime).

Die Inkubation bei 28 °C dauert bei Candida 3 Tage, bei allen übrigen Mikroorganismen 7 Tage.

Die Hemmkonzentration (MIC in µ/ml) wurde an folgenden Pilzen geprüft:

| | | |
|---|---|---|
| SA | = | Staphylococcus aureus 10 B |
| CA | = | Candida albicans ATCC 11651 |
| TG | = | Torulopsis glabrata K-589 |
| BD | = | Blastomyces derm. K-1573 |
| TQ | = | Trichophyton quinckeanum D-24 |
| MC | = | Microsporum canis ATCC-10214 |
| SS | = | Sporotrichum schenckii ATCC-10212 |

| Verb. Nr. | SA | CA | TG | BD | TQ | MC | SS |
|---|---|---|---|---|---|---|---|
| 1.1 | 32 | 4 | 8 | 16 | 4 | 8 | 8 |
| 1.9 | 64 | 2 | 32 | 4 | 2 | 32 | 32 |
| 1.14 | 64 | 4 | 32 | 8 | 2 | 8 | 32 |
| 1.3 | 64 | 16 | 64 | 16 | 2 | 4 | 64 |
| 1.26 | 32 | 4 | 8 | 8 | 1 | 16 | 16 |
| 1.28 | 64 | 4 | 16 | 8 | 1 | 16 | 16 |

**Patentansprüche**

1. Verbindungen der Formel I,

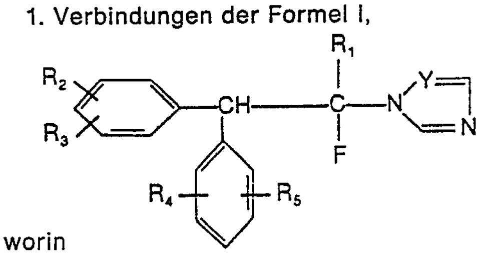

(I)

worin
Y für –CH= oder –N= steht; $R_1$ Wasserstoff oder $C_1$–$C_3$-Alkyl bedeutet; und $R_2$, $R_3$, $R_4$ und $R_5$ unabhängig voneinander für Wasserstoff, Halogen, Methyl, Methoxy, $CF_3$, $OCF_3$, $OCHF_2$, Cyano und/oder unsubstituiertes oder durch Fluor, Chlor und/oder Brom substituiertes Phenoxy stehen.

2. Verbindungen der Formel I nach Anspruch 1, worin Y für –N= steht; $R_1$ Wasserstoff oder $C_1$–$C_3$-Alkyl bedeutet; $R_2$ für Wasserstoff, 2–F, 2–Cl, 2–$CH_3$, 2–$C_2H_5$ oder 2–$CF_3$ steht; $R_3$ Wasserstoff, 4–Cl, 4–$OCHF_2$, 4-Phenoxy oder 4-(4'-Halophenoxy) ist; $R_4$ Wasserstoff, 2–F, 2–Cl, 2–$CH_3$ 2–$C_2H_5$ oder 2–$CF_3$ bedeutet; und $R_5$ für Wasserstoff oder 4–Cl, 4–F, 4–$OCHF_2$, 4-Phenoxy oder 4-(4'-Halophenoxy) steht.

3. Verbindungen der Formel I nach Anspruch 1, worin Y für –N= steht; $R_1$ Wasserstoff bedeutet, $R_2$ für Wasserstoff, 2–Cl oder 2–$CH_3$ steht; $R_3$ p-Halophenoxy bedeutet, $R_4$ Wasserstoff ist und $R_5$ für p-Halogen steht.

4. Eine Verbindung der Formel I nach Anspruch 1, ausgewählt aus der Reihe:
1-Fluor-1-(H-1,2,4-triazol-1-yl)-2-(2,4-dichlorphenyl)-2-(4-fluorphenyl)ethan;
1-Fluor-1-(1H-imidazol-1-yl)-2-(2,4-dichlorphenyl)-2-(4-fluorphenyl)ethan;
1-Fluor-1-(1H-1,2,4-triazol-1-yl)-2-bis(4-chlorphenyl)ethan;
1-Fluor-1-(1H-1,2,4-triazol-1-yl)-2-(4-chlorphenyl)-2-(4-fluorphenyl)ethan;
1-Fluor-1-(1H-1,2,4-triazol-1-yl)-2-(4-chlorphenyl)-2-(2-fluorphenyl)ethan;
1-Fluor-1-(1H-1,2,4-triazol-1-yl)-2-phenyl-2(2,4-dichlorphenyl)ethan.

5. Verfahren zur Herstellung einer Verbindung der Formel I nach Anspruch 1, dadurch gekennzeichnet, daß man einen Alkohol der Formel II

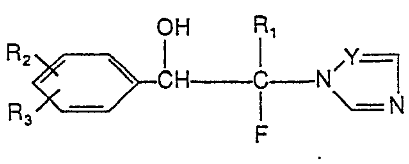

(II)

in Gegenwart einer Säure oder Lewis-Säure mit einem Benzolderivat der Formel III

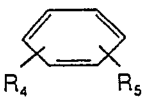

(III)

umsetzt, wobei $R_1$, $R_2$, $R_3$, $R_4$, $R_5$ und Y die unter Formel I angegebenen Bedeutungen haben.

6. Mikrobizides Mittel zur Bekämpfung oder Verhütung eines Befalls durch Mikroorganismen, dadurch gekennzeichnet, daß es als mindestens eine aktive Komponente eine Verbindung der Formel I gemäß Anspruch 1, zusammen mit üblichen Formulierungshilfsstoffen enthält.

7. Mittel gemäß Anspruch 6, dadurch gekennzeichnet, daß es als mindestens eine aktive Komponente eine Verbindung der Formel I gemäß Anspruch 2 enthält.

8. Mittel gemäß Anspruch 6, dadurch gekennzeichnet, daß es als mindestens eine aktive Komponente eine Verbindung der Formel I gemäß Anspruch 3 enthält.

9. Mittel gemäß Anspruch 6, dadurch gekennzeichnet, daß es als mindestens eine aktive Komponente eine Verbindung der Formel I gemäß Anspruch 4 enthält.

10. Mittel nach Anspruch 6, dadurch gekennzeichnet, daß es 0,1 bis 99% eines Wirkstoffs der Formel I, 99,9 bis 1% eines festen oder flüssigen Zusatzstoffes und 0 bis 25% eines Tensides enthält.

11. Mittel nach Anspruch 10, dadurch gekennzeichnet, daß es 0,1 bis 95% eines Wirkstoffes der Formel I, 99,8 bis 5% eines festen oder flüssigen Zusatzstoffes und 0,1 bis 25% eines Tensides enthält.

12. Verfahren zur Bekämpfung oder Verhütung eines Befalls von Kulturpflanzen durch phytopathogene Mikroorganismen, dadurch gekennzeichnet, daß man eine Verbindung der Formel I gemäß Anspruch 1 auf die Pflanze oder deren Standort appliziert.

13. Verwendung von Verbindungen der Formel I gemäß Anspruch 1 zur Bekämpfung und/oder zur präventiven Verhütung eines Befalls von Kulturpflanzen durch phytopathogene Mikroorganismen.

14. Verwendung gemäß Anspruch 13 von Verbindungen der Formel I gemäß einem der Ansprüche 2 bis 4.

15. Verwendung gemäß Anspruch 14, dadurch gekennzeichnet, daß es sich bei den Mikroorganismen um phytopathogene Pilze handelt.

16. Verwendung gemäß Anspruch 14, dadurch gekennzeichnet, daß es sich bei den Mikroorganismen um phytopathogene Pilze handelt.

**Claims**

1. Compounds of the formula I

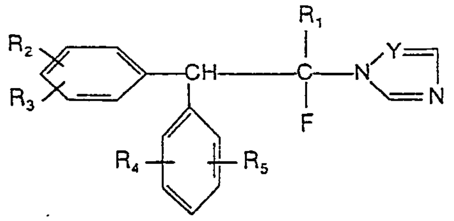

(I)

wherein Y is –CH= or –N=, $R_1$ is hydrogen or $C_1$–$C_3$alkyl, and $R_2$, $R_3$, $R_4$ and $R_5$ independently of one another are each hydrogen, halogen, methyl, methoxy, $CF_3$, $OCF_3$, $OCHF_2$, cyano, and/or phenoxy which is unsubstituted or substituted by fluorine, chlorine and/or bromine.

2. Compounds of the formula I according to claim 1, wherein Y is –N=, $R_1$ is hydrogen or $C_1$–$C_3$alkyl, $R_2$ is hydrogen, 2–F, 2–Cl, 2–$CH_3$, 2–$C_2H_5$ or 2–$CF_3$, $R_3$ is hydrogen, 4–Cl, 4–$OCHF_2$, 4-phenoxy or 4-(4′-halophenoxy), $R_4$ is hydrogen, 2–F, 2–Cl, 2–$CH_3$, 2–$C_2H_5$ or 2–$CF_3$, and $R_5$ is hydrogen, 4–Cl, 4–F, 4–$OCHF_2$, 4-phenoxy or 4-(4′-halophenoxy).

3. Compounds of the formula I according to claim 1, wherein Y is –N=, $R_1$ is hydrogen, $R_2$ is hydrogen, 2–Cl or 2–$CH_3$, $R_3$ is p-halophenoxy, $R_4$ is hydrogen, and $R_5$ is p-halogen.

4. A compound of the formula I according to claim 1, selected from the series comprising:
1-fluoro-1-(1H-1,2,4-triazol-1-yl)-2-(2,4-dichlorophenyl)-2-(4-fluorophenyl)ethane,
1-fluoro-1-(1H-imidazol-1-yl)-2-(2,4-dichlorophenyl)-2-(4-fluorophenyl)ethane,
1-fluoro-1-(1H-1,2,4-triazol-1-yl)-2-bis(4-chlorophenyl)ethane,
1-fluoro-1-(1H-1,2,4-triazol-1-yl)-2-(4-chlorophenyl)-2-(4-fluorophenyl)ethane,
1-fluoro-1-(1H-1,2,4-traizol-1-yl)-2-(4-chlorophenyl)-2-(2-fluorophenyl)ethane and
1-fluoro-1-(1H-1,2,4-triazol-1-yl)-2-phenyl-2-(2,4-dichlorophenyl)ethane.

5. A process for producing a compound of the formula I according to claim 1, characterised in that an alcohol of the formula II

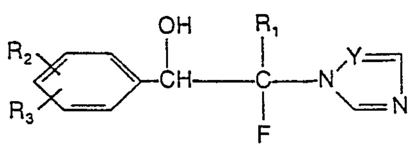

(II)

is reacted, in the presence of an acid or Lewis acid, with a benzene derivative of the formula III

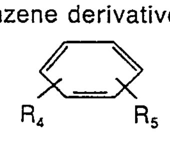

(III)

wherein $R_1$, $R_2$, $R_3$, $R_4$, $R_5$ and Y have the meanings defined under the formula I.

6. A microbicidal composition for controlling or preventing an infestation by microorganisms, characterised in that it contains, as at least one active component, a compound of the formula I according to claim 1, together with customary formulation auxiliaries.

7. A composition according to claim 6, characterised in that it contains, as at least one active component, a compound of formula I as claimed in claim 2.

8. A composition according to claim 6, characterised in that it contains, as at least one active component, a compound of formula I as claimed in claim 3.

9. A composition according to claim 6, characterised in that it contains, as at least one active component, a compound of formula I as claimed in claim 4.

10. A composition according to claim 6, characterised in that it contains 0.1 to 99% of an active ingredient of the formula I, 99.9 to 1% of a solid or liquid additive, and 0 to 25% of a surfactant.

11. A composition according to claim 10, characterised in that it contains 0.1 to 95% of an active ingredient of the formula I, 99.8 to 5% of a solid or liquid additive, and 0.1 to 25% of a surfactant.

12. A process for controlling or preventing an infestation of cultivated plants by phytopathogenic microorganisms, characterised in that a compound of the formula I according to claim 1 is applied to the plant or to the locus thereof.

13. The use of compounds of the formula I according to claim 1 for controlling and/or preventing an infestation of cultivated plants by phytopathogenic microorganisms.

14. The use according to claim 13 of compounds of the formula I according to any one of claims 2 to 4.

15. The use according to claim 14, characterised in that the microorganisms concerned are phytopathogenic fungi.

16. The use according to claim 14, characterised in that the microorganisms concerned are phytopathogenic fungi.

**Revendications**

1. Composés de formule I

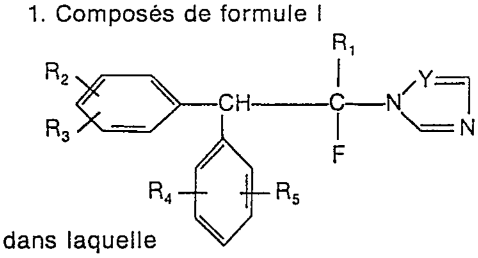

(I)

dans laquelle

Y représente –CH= ou –N=; $R_1$ représente l'hydrogène ou un groupe alkyle en $C_1$–$C_3$; et $R_2$, $R_3$, $R_4$ et $R_5$ représentent chacun, indépendamment les uns des autres, l'hydrogène, un halogène, un groupe méthyle, méthoxy, $CF_3$, $OCF_3$, $OCHF_2$, cyano et/ou un groupe phénoxy non substitué ou substitué par le fluor, le chlore et/ou le brome.

2. Composé de formule I selon la revendication 1, dans lesquels Y représente –N=; $R_1$ représente l'hydrogène ou un grupe alkyle en $C_1$–$C_3$; $R_2$ représente l'hydrogène, un substituant 2–F, 2–Cl, 2–$CH_3$, 2–$C_2H_5$ ou 2–$CF_3$; $R_3$ représente l'hydrogène, un substituant 4–Cl, 4–$OCHF_2$, 4-phénoxy ou 4-(4′-halogénophénoxy); $R_4$ représente l'hydrogène, un substituant 2–F, 2–Cl, 2–$CH_3$, 2–$C_2H_5$ ou 2–$CF_3$; et $R_5$ représente l'hydrogène ou un substi-

tuant 4–Cl, 4–F, 4–$OCHF_2$, 4-phénoxy ou 4-(4′-halogénophénoxy).

3. Composés de formule I selon la revendication 1, dans lesquels Y représente –N=; $R_1$ représente l'hydrogène, $R_2$ représente l'hydrogène, un substituant 2–Cl ou 2–$CH_3$; $R_3$ représente un groupe p-halogénophénoxy, $R_4$ l'hydrogène et $R_5$ un substituant p-halogéno.

4. Un composé de formule I selon la revendication 1, choisi dans le groupe suivant:

1-fluoro-1-(1H-1,2,4-triazole-1-yl)-2-(2,4-dichlorophényl)-2-(4-fluorophényl)-éthane;

1-fluoro-1-(1H-imidazole-1-yl)-2-(2,4-dichlorophényl)-2-(4-fluorophényl)-éthane;

1-fluoro-1-(1H-1,2,4-triazole-1-yl)-2-bis-(4-chlorophényl)-éthane;

1-fluoro-1-(1H-1,2,4-triazole-1-yl)-2-(4-chlorophényl)-2-(4-fluorophényl)-éthane;

1-fluoro-1-(1H-1,2,4-triazole-1-yl)-2-(4-chlorophényl)-2-(2-fluorophényl)-éthane;

1-fluoro-1-(1H-1,2,4-triazole-1-yl)-2-phényl-2-(2,4-dichlorphényl)-éthane.

5. Procédé de préparation d'un composé de formule I selon la revendication 1, caractérisé en ce que l'on fait réagir un alcool de formule II

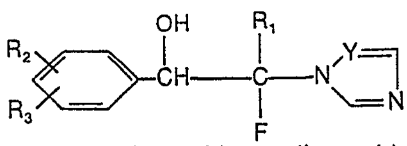

(II)

en présence d'un acide ou d'un acide de Lewis avec un dérivé du benzène répondant à la formule III

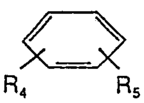

(III)

$R_1$, $R_2$, $R_3$, $R_4$, $R_5$ et Y ayant les significations indiquées en référence à la formule I.

6. Produit microbicide pour combattre ou prévenir une attaque par des microorganismes, caractérisé en ce qu'il contient au moins un composant actif consistant en un composé de formule I selon la revendication 1, avec des produits auxiliaires de formulation usuels.

7. Produit selon la revendication 6, caractérisé en ce qu'il contient au moins un composant actif consistant en un composé de formule I selon la revendication 2.

8. Produit selon la revendication 6, caractérisé en ce qu'il contient au moins un composant actif consistant en un composé de formule I selon la revendication 3.

9. Produit selon la revendication 6, caractérisé en ce qu'il contient au moins un composant actif consistant en un composé de formule I selon la revendication 4.

10. Produit selon la revendication 6, caractérisé en ce qu'il contient de 0,1 à 99% d'une substance active de formule I, de 99,9 à 1% d'un additif solide ou liquide et de 0 à 25% d'un agent tensioactif.

11. Produit selon la revendication 10, caractérisé en ce qu'il contient de 0,1 à 95% d'une substance active de formule I, de 99,8 à 5% d'un additif solide ou liquide et de 0,1 à 25% d'un agent tensioactif.

12. Procédé pour combattre ou prévenir une attaque de végétaux cultivés par des microorganismes phytopathogènes, caractérisé en ce que l'on applique un composé de formule I selon la revendication 1 sur la plante ou son habitat.

13. Utilisation des composés de formule I selon la revendication 1, pour combattre et/ou prévenir une attaque de végétaux cultivés par des microorganismes phytopathogènes.

14. Utilisation selon la revendication 13 de composés selon l'une des revendications 2 à 4.

15. Utilisation selon la revendication 14, caractérisée en ce que les microorganismes sont des mycètes phytopathogènes.

16. Utilisation selon la revendication 14, caractérisée en ce que les microorganismes sont des mycètes phytopathogènes.